# EUROPÄISCHE PATENTSCHRIFT

(11) **EP 0 677 283 B1**
(45) Veröffentlichungstag und Bekanntmachung des Hinweises auf die Patenterteilung: **30.01.2002**
(21) Anmeldenummer: 95104775.2
(22) Anmeldetag: 31.03.1995
(51) Int. Cl.: A61F 7/00, A61G 13/10, H05B 3/34, A61B 6/04

(54) **Medizinische Wärmematte**
Medical heating mat
Tapis chauffant médical

(30) Priorität: 14.04.1994 DE 4412471
(43) Veröffentlichungstag der Anmeldung: 18.10.1995
(73) Patentinhaber: Schneider, Klaus, 71126 Gäufelden (DE)
(72) Erfinder: Schneider, Klaus, 71126 Gäufelden (DE)
(74) Vertreter: Weller, Wolfgang, Dr.rer.nat.

(56) Entgegenhaltungen:
- EP-A- 0 152 592
- WO-A-89/06931
- BE-A- 688 786
- DE-A- 2 743 768
- DE-A- 3 146 234
- DE-A- 3 339 781
- US-A- 2 504 697

## Beschreibung

Die Erfindung betrifft eine flexible medizinische Wärmematte zum Wärmen von Patienten, mit einer Matte, in der eine elektrische Widerstandsheizung integriert ist, wobei die Heizung ein stromleitendes Material aufweist, das in einer solchen Menge vorhanden ist, daß Röntgenstrahlung ohne Reflexion das Material durchlaufen kann, wobei ferner die Matte aus Silikon besteht, das das stromleitende Material umhüllt.

Eine Wärmematte der eingangs genannten Art ist aus der WO 89-A-06931 bekannt.

Derartige medizinische Wärmematten dienen dazu, Patienten, die auf einer solchen Wärmematte liegen, zu wärmen. Einsatzbereiche sind beispielsweise Operationstische oder Krankenbetten. Derartige Matten können als Matratzenauflage in Aufwachräumen und Intensivstationen eingesetzt werden. Gleichermaßen können derartige medizinische Wärmematten Einsatz in REHA-Zentren oder bei der Heilgymnastik finden, beispielsweise zur Muskelentspannung bei Übungen mit Patienten mit Querschnittslähmungen.

Bei der aus der eingangs genannten WO 89-A-06931 bekannten medizinischen Wärmematte besteht die elektrische Widerstandsheizung aus einem mit einem bestimmten elektrischen Widerstand behafteten Leiter, der auf einen Träger schraubenlinienförmig aufgewickelt ist. Der schraubenlinienförmig gewickelte Heizdraht ist in einem Materialverbund aus mehreren Schichten eingebettet, der durch eine Vulkanisation der Schichten erreicht wird. Insgesamt besteht die Heizauflage aus vier Schichten, von denen drei Schichten aus einer Silikon-Kautschuk-Mischung bestehen, während die vierte Schicht eine Gewebeschicht ist. Die Silikon-Kautschuk-Schichten weisen untereinander unterschiedliche Weichheitsgrade auf, wobei die äußerste Silikon-Kautschuk-Schicht der Heizauflage die Weichheit der Heizauflage bestimmt. Die Gewebeschicht, die aus einer Matte aus Glasfasern besteht, grenzt die gummielastischen Eigenschaften der Silikon-Kautschuk-Schichten ein.

Eine medizinische Wärmematte soll einen menschlichen Körper, der auf der Wärmematte liegt, auf Körpertemperatur halten, demzufolge soll die Temperatur der Wärmematte etwa im Bereich der Körpertemperatur, also um 36°C, liegen.

Bei der elektrischen Widerstandsheizung in Form von Drähten ist keine gleichmäßige Wärmeverteilung zu erzielen, da in der Matte aufgrund der drahtförmigen Heizelemente, im Schnitt durch einen Heizdraht gesehen, eine punktförmige Wärmeabgabe vorherrscht, die in unmittelbarer Umgebung des Drahtes für eine relativ hohe Wärme sorgt, zwischen zwei benachbarten Heizdrähten jedoch ein abnehmender Wärmegradient zu spüren ist. Ein auf einer derartigen Wärmematte liegender Patient spürt eine derartige punktuelle oder linienförmige Wärmeabgabe, so daß keine an das Wärmeempfindungssystem des menschlichen Körpers angepaßte gleichmäßige Wärme durch eine solche Matte zur Verfügung gestellt werden kann. Reizungen durch die punktuelle höhere Temperatur im Kabelbereich und Nervenzuckungen sind nicht auszuschließen.

Die aus metallischem Material bestehenden Heizdrähte sind bei Röntgenaufnahmen, die von einem Patienten angefertigt werden, der auf einer derartigen Wärmematte liegt, zu erkennen. Der Arzt muß bei der Auswertung einer derartigen Röntgenaufnahme für die Diagnose die deutlich erkennbaren Heizdrähte wegdenken, was bei manchen Diagnosen, die beispielsweise auf dem Erkennen von Rissen oder dergleichen beruhen, zu großen Schwierigkeiten führen kann. Zum Anfertigen von Röntgenaufnahmen ist es aber oftmals deswegen erforderlich, den Patienten auf eine medizinische Wärmematte zu legen, da, falls der Patient vorher auf einer Wärmematte geruht hat und nun zur Röntgenaufnahme auf eine "kalte" Unterlage gebracht wird, er dies spürt und mit Unruhe reagiert, so daß dann keine korrekte Röntgenaufnahme durchgeführt werden kann.

Aufgrund der schraubenlinienförmig aufgewickelten Heizdrähte weist die Wärmematte eine erhebliche Dicke auf, die das Handling dieser transportablen Wärmematte erschwert.

Aus der US-A-2 504 697 ist ein Röntgentisch bekannt, bei dem die Patientenauflage beheizbar ist. In dem Röntgentisch ist dazu eine elektrische Widerstandsheizung eingebettet, wobei der Heizleiter aus einer Aluminiumfolie besteht. Bei diesem Röntgentisch ist allerdings die Auflage, auf der der Patient bei der Röntgenuntersuchung liegt, eben und hart, so daß sich die Unterlage nicht an die Haut des Patienten anschmiegt, und ist ferner nicht transportabel.

Weiterhin ist aus der DE-A-33 39 781 ein Röntgendiagnostikgerät bekannt, das eine harte Patientenlagerungsplatte aufweist, die aus Kohlenstoffasermaterial besteht. An der Patientenlagerungsplatte ist eine Heizstromquelle angeschlossen, die durch das Kohlenstoffasermaterial einen Heizstrom zur Beheizung der Lagerfläche schickt.

Ein weiteres Röntgengerät mit einer harten Patientenlagerungsplatte ist aus der EP-A-0 152 592 bekannt, bei dem in der Patientenlagerungsplatte eine elektrische Heizvorrichtung unmittelbar unter der Deckplatte angeordnet ist. Diese kann von einem metallisierten Gewebe, einer Leitlackschicht oder einer dünnen Metallschicht auf der Rückseite der Deckplatte der in Sandwichbauweise aufgebauten Patientenlagerungsplatte gebildet sein.

Aus der DE-A-688 786 ist ein beheizbarer Röntgentisch offenbart, dessen den Patienten tragende Auflage ebenfalls fest mit dem Röntgentisch verbunden ist, d.h. nicht für andere Einsatzzwecke geeignet ist. Die Patientenlagerungsplatte besteht aus einem harten ebenen Kern, auf dem mehrere dünne Schichten aus mit Harz imprägniertem Hartpapier und aus mit Harz imprägnierten Gewebeschichten angeordnet sind, die insgesamt zu einer wiederum harten Patientenauflage führen. Die elektrische Widerstandsheizung dieses bekannten Röntgentisches besteht aus einer mehrschichtigen Anordnung aus kohlenstoffhaltigem Material sowie einer oder mehreren Asbestschichten.

Ferner ist in der DE-A-31 46 234 ein vernickeltes textiles Flächengebilde als für Röntgenstrahlen durchlässiges Heizgewebe offenbart, das zur Heizung von Operationstischen einsetzbar ist, während in der DE-A-27 43 768 ein metallisiertes Textilmaterial offenbart ist, das als Material für Flächenheizelemente verwendbar ist.

Es ist Aufgabe der vorliegenden Erfindung, eine flexible medizinische Wärmematte der eingangs genannten Art dahingehend zu verbessern, daß zum einen für eine gleichmäßigere Wärmeverteilung in der Matte gesorgt wird, wobei zum anderen es möglich ist, Röntgenaufnahmen durch eine derartige Wärmematte hindurch zu fertigen, ohne daß dies in der Röntgenaufnahme zu erkennen ist, und daß die Wärmematte besonders leicht zu handhaben ist, insbesondere zu verschiedenen Einsatzzwecken verwendbar ist, und daß der Patient das Liegen auf der Wärmematte als besonders angenehm empfindet.

Erfindungsgemäß wird die Aufgabe dadurch gelöst, daß das stromleitende Material etwa über die gesamte Fläche der Matte flächig ausgebildet ist, und daß der Härtegrad des Silikons zwischen 30-40 beträgt.

Dadurch, daß das stromleitende Material der Heizung nun nicht mehr drahtförmig, sondern als flächiges Material ausgebildet ist, kann eine über die Fläche der Wärmematte gesehen gleichmäßige Wärmeverteilung erzielt werden. Ferner wird errreicht, daß Röntgenstrahlung ohne Reflexionen durch das Material hindurchtreten kann, so daß eine Röntgenaufnahme, die durch eine solche Wärmematte hindurch angefertigt wurde, keinerlei Signale von der Heizung der Matte zeigt.

Durch die flächige Verteilung des stromleitenden Materials ist es nicht nur möglich, die gleichmäßige Wärmeverteilung besonders günstig zu erreichen, sondern das Heizmaterial kann entsprechend dünn bzw. in einer solchen geringen Menge pro Flächen- bzw. Volumeneinheit einer solchen Wärmematte vorhanden sein, daß Röntgenstrahlungen zum Anfertigen von medizinischen Röntgenaufnahmen ohne Reflexion das Material durchlaufen können.

Für einen Patienten, der auf einer erfindungsgemäßen medizinischen Wärmematte liegt, ergibt sich durch die gleichmäßig verteilte flächenförmige Wärmeabgabe ein wesentlich angenehmeres Wärmeübertragungssystem, das dem menschlichen Wärmeempfindungssystem angepaßt ist, das nämlich auf flächig gleichmäßig verteilte Wärmeangebote angenehmer reagiert als auf punkt- oder linienförmige Wärmeangebote.

Für den Arzt, der eine Röntgenaufnahme von einem Patienten anfertigt, der auf einer erfindungsgemäßen Wärmematte liegt, ist es nun nicht mehr notwendig, daß dieser gedanklich von dem angefertigten Röntgenbild Heizdrähte, Fühler oder Kabelverbindungen abziehen muß.

Es ist nun beispielsweise möglich, sowohl in einem Krankenbett als auch auf einer Röntgenunterlage eine derartige erfindungsgemäße Wärmematte vorzusehen, so daß es nur noch notwendig ist, den Patienten kurz von einem Bett auf die Röntgenunterlage zu überbringen, wobei auf letzterer er dasselbe angenehme gleichmäßige Wärmegefühl hat wie in einem Bett, so daß dieses angenehme Gefühl den Patienten beruhigt und er sich entsprechend ruhig während der Röntgenaufnahme verhält. Dadurch ist dann auch die Notwendigkeit eventueller Zweitaufnahmen wegen Bewegungen des Patienten während der Aufnahme auszuschließen.

Durch die Auswahl von Silikon wird sowohl die Ausgestaltung sehr flexibler Wärmematten ermöglicht, als auch zugleich sehr widerstands- und strapazierfähiger Matten ermöglicht, die in dem alltäglichen Krankenhausbetrieb auf Dauer einsetzbar sind.

Dadurch, daß der Härtegrad der Silikonumhüllung zwischen 30-40 beträgt, entspricht der Härtegrad der Silikonumhüllung demjenigen der menschlichen Haut, wodurch ein Patient, der auf einer derartigen Matte liegt, ein besonders angenehmes Gefühl auf der Haut verspürt, d.h. es wird ihm eine Wärme vermittelt, wie dies beispielsweise einem direkten Hautkontakt mit einem anderen menschlichen Körper entspricht. Dies hat auch eine psychologische heilfördernde Wirkung, so wird beispielsweise Kleinkindern unterschwellig das Gefühl vermittelt, als stünden sie in direktem Hautkontakt mit dem wärmenden Körper eines Elternteils.

Somit wird die Aufgabe vollkommen gelöst.

In einer weiteren Ausgestaltung der Erfindung ist das stromleitende Material als Bestandteil einer Heizfolie ausgebildet.

Heizfolien sind relativ dünne, flächige Gebilde, die bislang auf anderen technischen Gebieten Einsatz gefunden haben, und durch die eine über den Flächenbereich der Folie gleichmäßige

Wärmeabstrahlung möglich ist. Es ist nunmehr dafür zu sorgen, daß solche Folien ausgewählt werden, die entsprechend röntgenstrahlendurchlässig sind, d.h. eine Röntgenaufnahme nicht beeinflussen.

In einer weiteren Ausgestaltung der Erfindung ist das stromleitende Material fein verteilt auf einem flächigen, flexiblen Träger aufgebracht.

Diese Maßnahme hat nun den erheblichen Vorteil, daß eine flexible Wärmematte erzielt wird, die sich den Körperrundungen hervorragend anpassen kann, so daß beispielsweise eine solche Matte auch um Körperteile, wie beispielsweise die Beine, gelegt werden kann, ohne eine Tendenz zu zeigen, sich in Ausrichtung von relativ steifen Heizdrähten zu entspannen.

In einer weiteren Ausgestaltung der Erfindung ist der flächige textile Träger ein Glasseidengewebe.

Diese Maßnahme hat nun den erheblichen Vorteil, daß auf eine isolierende Basis in Form eines Glasseidengewebes Materialien aufgetragen werden können, die entsprechend der gewünschten Heizleistung in ausreichender Konzentration, zugleich aber auch in solch geringen Mengen gezielt aufgetragen werden können, um sicherzustellen, daß eine Röntgenaufnahme nicht beeinflußt wird. Zugleich wird durch das Gewebe eine besonders geschmeidige Flexibilität der Heizung in der Wärmematte erreicht.

In einer weiteren Ausgestaltung der Erfindung ist das stromleitende Material eine Silber-Kohlenstaub-Mischung.

Ein derartiges Material als stromleitendes Material kann großtechnisch sehr gut verarbeitet werden und den jeweiligen Bedürfnissen angepaßt werden, d.h. also ausreichende Heizleistung mit gleichzeitiger Sicherstellung, daß keine solche Materialanhäufung pro Flächen- bzw. Volumeneinheit vorherrscht, daß Röntgenstrahlen beeinträchtigt werden.

In einer weiteren Ausgestaltung der Erfindung ist das flächige stromleitende Material beidseitig mit einem isolierenden flexiblen Kunststoff bedeckt.

Diese Maßnahme hat nun den Vorteil, daß die Heizung, die in der Matte aufgenommen ist, ein nach außen hin nicht leitendes abgeschlossenes System darstellt, so daß auch äußere Verletzungen der Matte keine Beeinträchtigung der Heizung nach sich ziehen können, insbesondere keine Korrosion oder dergleichen.

In einer weiteren Ausgestaltung der Erfindung weist die Matte eine glatte, porenfreie Oberfläche auf.

Diese Maßnahme hat den Vorteil, daß austretende Körperflüssigkeiten leicht abgewischt werden können und somit eine Keimbildung ausgeschlossen wird. Die Wärmematte ist dadurch bakterizid. Zugleich ist sie durch die glatte Oberfläche einfach zu handhaben und auch einfach desinfizierbar.

In einer weiteren Ausgestaltung der Erfindung ist die Matte mit einem Steuergerät verbunden, über das das stromleitende Material elektrisch versorgbar und parametrierbar ist.

Diese Maßnahme hat nun den Vorteil, daß die Stromversorgungs- und Steuerelemente abseits der Matte angeordnet sind, so daß, außer einem Verbindungskabel zwischen Steuergerät und Matte, keinerlei Materialien eingesetzt werden müssen, die in einer Röntgenaufnahme erscheinen.

In einer weiteren Ausgestaltung der Erfindung ist das stromleitende Material mit Niedervoltspannung betreibbar.

Diese Maßnahme hat nun den erheblichen Vorteil, daß durch die Niedervoltspannung ein beachtlicher Sicherheitsaspekt gewährleistet ist. Wird beispielsweise an einem Patienten, der auf einer derartigen Matte liegt, eine Manipulation mit einem Schneide- oder Stichgerät vorgenommen, und wird mit diesem Gerät beispielsweise beim Beiseitelegen versehentlicherweise in die Matte geschnitten, wird ein elektrischer Kurzschluß, der entweder den Patienten oder den Arzt beeinträchtigen könnte, ausgeschlossen, da derartige niedere Spannungen keinerlei Spannungsschocks auslösen können.

In einer weiteren Ausgestaltung der Erfindung sind mehrere Heizfolien übereinander geschichtet vorgesehen.

Diese Maßnahme hat den Vorteil, daß zum einen eine besonders gleichmäßige Wärmeabstrahlung deswegen erzielt werden kann, da über die Fläche einer Folie durch Fertigungstoleranzen vorhandene Wärmeabstrahlgradienten ausgeglichen werden können, und daß zum anderen unterschiedliche Wärmeleitfähigkeiten von unterschiedlichen Mattenmaterialien bzw. Dicken von Matten durch Aufeinanderlegen mehrerer Heizungsfolien kompensiert werden können.

Es versteht sich, daß die vorstehend genannten und die nachstehend noch zu erläuternden Merkmale nicht nur in den angegebenen Kombinationen sondern auch in anderen Kombinationen oder in Alleinstellung einsetzbar sind, ohne den Rahmen der vorliegenden Erfindung zu verlassen.

Die Erfindung wird nachfolgend anhand eines ausgewählten Ausführungsbeispiels im Zusammenhang mit den beiliegenden Zeichnungen näher beschrieben und erläutert.

Es zeigen:
- Fig. 1: stark schematisiert einen Schnitt durch eine Mattenebene einer erfindungsgemäßen Wärmematte,
- Fig. 2: einen Schnitt längs der Linie II-II in Fig. 1, und
- Fig. 3: eine ausschnittsweise stark vergrößerte Schnittdarstellung längs der Linie III in Fig. 1.

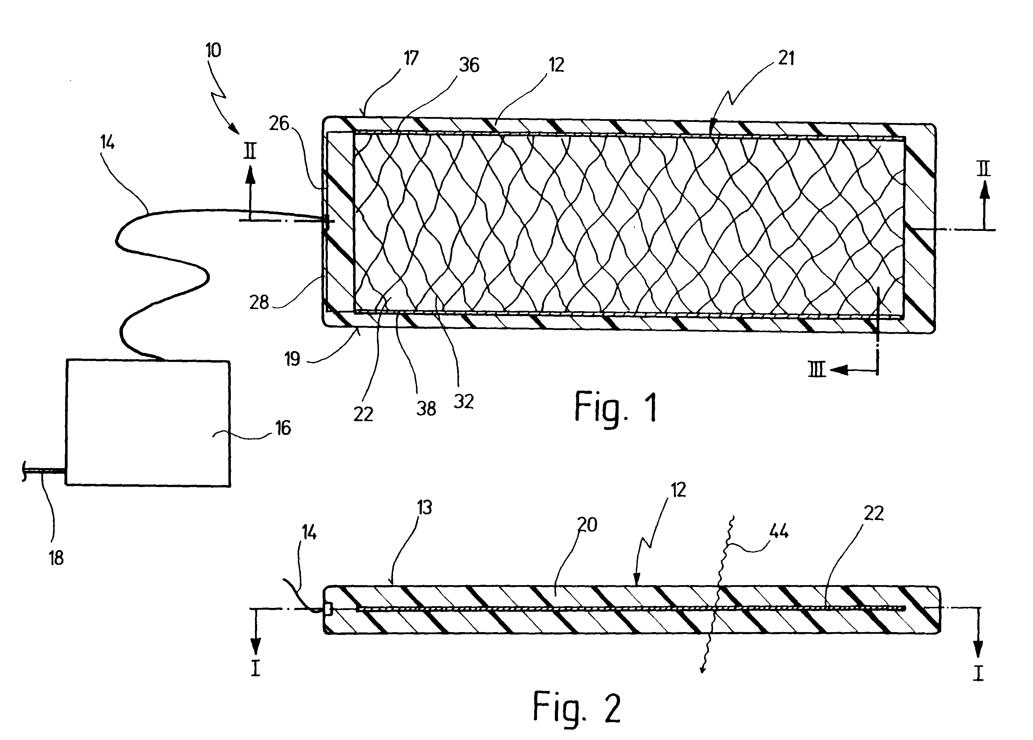

In den Figuren 1 bis 3 ist eine erfindungsgemäße medizinische Wärmematte in ihrer Gesamtheit mit der Bezugsziffer 10 versehen.

Die Wärmematte 10 weist eine Matte 12 auf, die über ein Kabel 14 elektrisch mit einem Steuergerät 16 verbunden ist.

Das Steuergerät 16 wiederum ist über ein Kabel 18 mit einer Stromquelle verbindbar.

Die Matte 12 des dargestellten Ausführungsbeispiels weist in etwa die Maße 500 mm x 1.500 mm x 7 mm auf und weist einen Körper 20 aus einem Silikonmaterial auf. Der Härtegrad der Silikonumhüllung entspricht weitgehend dem Härtegrad der menschlichen Haut, also zwischen 30 - 40 Härtegraden.

Im Körper 20 aus Silikon ist eine Heizung 21 mittig eingebettet.

Die Heizung 21 besteht aus einer sich in einer etwa mittigen Ebene (siehe insbesondere Fig. 2) erstreckenden Heizfolie 22.

Die Heizfolie 22 weist in Draufsicht eine etwa rechteckförmige Form auf und erstreckt sich zwischen den gegenüberstehenden Längskanten 17 und 19 der Matte 12 sowie etwa über deren Länge. An gegenüberliegenden Längskanten ist die Heizfolie 22 mit Kontaktstreifen 36 und 38 versehen.

Endseitige Leitungen 26 und 28, die mit den Kontaktstreifen 36 und 38 verbunden sind, werden voneinander elektrisch isoliert zu dem Kabel 14 vereint zum Steuergerät 16 geführt.

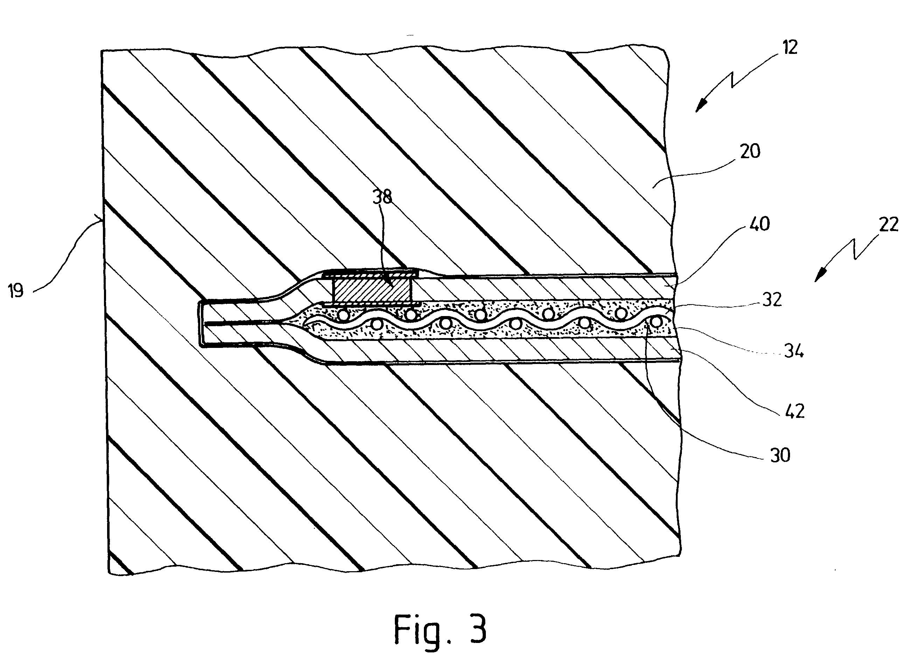

Aus der vergrößerten Schnittdarstellung von Fig. 3 ist zu erkennen, daß die Heizfolie 22 als Basis einen Träger 30 in Form eines Gewebes 32 aufweist.

Das Gewebe 32 besteht aus einem Glasseidengewebe.

Das Gewebe 32 ist beidseitig mit einem stromleitenden Material 34 beschichtet, das das Gewebe auch durchdringt.

Das stromleitende Material 34 besteht aus einer Silber-Kohlenstaub-Mischung.

Das stromleitende Material 34 ist beidseitig mit einer Kunststoffolie, hier einer Polyesterfolie 40, 42, kaschiert. Seitlich stehen die Folien 40 und 42 über das Material 34 hinaus und sind miteinander verbunden.

Die Kontaktstreifen 36 bzw. 38 bestehen aus einem verzinnten Kupferband. Über die Kontaktstreifen kann das stromleitende Material 34 mit einer Stromquelle verbunden werden.

Die Stromführung ist nun derart, daß von einem Heiztransformator des Steuergerätes 16 eine Ausgangsspannung von 24 Volt zur Verfügung gestellt wird und beispielsweise über die Leitung 28 dem entsprechenden Kontaktstreifen 38 der Heizfolie 22 zugeführt wird. Der elektrische Strom wird gleichmäßig verteilt über das stromleitende Material 34 flächig zur gegenüberliegenden Seite der Heizfolie 22 zum Kontaktstreifen 36 geführt, und der Stromkreis wird über die Leitung 26 geschlossen.

Die Heizleistung der im Ausführungsbeispiel dargestellten Wärmematte 10 liegt bei etwa 130 Watt mit einer maximalen Stromaufnahme von etwa 500 mA.

Mit dieser Ausgestaltung kann durch die Matte 12 Röntgenstrahlung 44, wie sie üblicherweise bei medizinischen Röntgenaufnahmen eingesetzt wird, durchtreten, ohne daß irgendwelche Reflexionen auf dem Röntgenbild ersichtlich sind.

Die Oberfläche 13 der Matte 12 ist glatt und porenfrei.

Aufgrund der gleichmäßigen flächigen Wärmeabgabe bei Stromfluß durch das stromleitende Material 34 der Heizfolie 22 wird eine flächige gleichmäßige Wärmeverteilung in der Matte 12 erreicht, d.h., ein Patient, der auf der Matte 12 liegt, kann gleichmäßig verteilt Wärme aufnehmen. Durch diese Maßnahme ist nunmehr die zwischenzeitlich anerkannte Methode zur Vermeidung intra-operativer Hypothermie gewährleistet.

Ein höchster Stand an Sicherheit in der Temperaturregelung kann dadurch erreicht werden, daß mehrere Temperatursensoren innerhalb der Heizmatte sich gegenseitig überwachen. Dabei können optische und akustische Alarmfunktionen vom Steuergerät 16 abgegeben werden. Dazu ist im Steuergerät 16 ein Trenntransformator zur Umwandlung in Schutzkleinspannung 24 Volt eingebaut. Am Steuergerät wird die Soll-Temperatur eingestellt und durch einfachen Knopfdruck in Betrieb gesetzt. Die Soll- und die Ist-Werte werden optisch angezeigt. Die letzten zwei Grad zur Soll-Temperatur werden getaktet nach oben zur Soll-Temperatur gefahren, so daß eine Überhitzung schon von der elektrischen Steuerung und den dadurch gegebenen Impulsen ausgeschlossen werden kann. Das Steuergerät 16 gibt bei Störungen optische und akustische Alarmsignale ab, so daß das Krankenhauspersonal jederzeit genauestens über die Funktion informiert ist. Eine Sicherheitsschaltung sorgt dafür, daß bei Erreichen einer Übertemperatur (z.B. im Störfall) von etwa 40°C die Heizung grundsätzlich abgeschaltet wird.

Die Matte 12 ist sehr flexibel und dadurch an den Körper anpaßbar, sie ist wasserdicht und desinfizierbar. Die Matte ist sehr schnell aufwärmbar, und die schnelle Aufwärmzeit garantiert auch bei einem kurzfristigen Einsatz einen größtmöglichen Komfort für den Patienten. Das körperfreundliche Silikonmaterial ist bakterizid, chemisch und mechanisch belastbar und auf Dauer temperaturbeständig.

## Patentansprüche

1. Flexible medizinische Wärmematte zum Wärmen von Patienten, mit einer Matte (12), in der eine elektrische Widerstandsheizung (21) integriert ist, wobei die Heizung (21) ein stromleitendes Material (34) aufweist, das in einer solchen Menge vorhanden ist, daß Röntgenstrahlung (44) ohne Reflexion das Material (34) durchlaufen kann, wobei ferner die Matte (12) aus Silikon (20) besteht, das das stromleitende Material (34) umhüllt, **dadurch gekennzeichnet, daß** das stromleitende Material (34) etwa über die gesamte Fläche der Matte (12) flächig ausgebildet ist, und daß der Härtegrad des Silikons (20) zwischen 30-40 beträgt.

2. Medizinische Wärmematte nach Anspruch 1, **dadurch gekennzeichnet, daß** das stromleitende Material (34) als Bestandteil einer Heizfolie (22) ausgebildet ist.

3. Medizinische Wärmematte nach Anspruch 1 oder 2, **dadurch gekennzeichnet, daß** das stromleitende Material (34) fein verteilt auf einem flächigen flexiblen Träger (30) aufgebracht ist.

4. Medizinische Wärmematte nach Anspruch 3, **dadurch gekennzeichnet, daß** der flächige flexible Träger (30) ein Glasseidengewebe (32) ist.

5. Medizinische Wärmematte nach einem der Ansprüche 1 bis 4, **dadurch gekennzeichnet, daß** das stromleitende Material (34) eine Silber-Kohlenstaub-Mischung ist.

6. Medizinische Wärmematte nach einem der Ansprüche 1 bis 5, **dadurch gekennzeichnet, daß** das flächige stromleitende Material (34) beidseitig mit einer isolierenden, flexiblen Kunststoffolie (40, 42) bedeckt ist.

7. Medizinische Wärmematte nach einem der Ansprüche 1 bis 6, **dadurch gekennzeichnet, daß** die Matte (12) eine glatte, porenfreie Oberfläche (13) aufweist.

8. Medizinische Wärmematte nach einem der Ansprüche 1 bis 7, **dadurch gekennzeichnet, daß** die Matte (12) mit einem Steuergerät (16) verbunden ist, über das das stromleitende Material (34) elektrisch versorgbar und parametrierbar ist.

9. Medizinische Wärmematte nach einem der Ansprüche 1 bis 8, **dadurch gekennzeichnet, daß** das stromleitende Material (34) mit Niedervoltspannung betreibbar ist.

10. Medizinische Wärmematte nach einem der Ansprüche 2 bis 9, **dadurch gekennzeichnet, daß** mehrere Heizfolien übereinander geschichtet vorgesehen sind.

## Claims

1. A flexible medical thermal mat for warming patients, comprising a mat (12) in which an electrical resistance heating (21) is integrated, wherein the heating (21) comprises a current conductive material (34) that is present in such an amount that X-rays (44) can pass the material (34) without reflection, wherein the mat (12) further consists of silicone (20) which covers the conductive material (34), **characterized in that** the conductive material (34) is flatly configured over approximately the complete area of the mat (12) and that the degree of hardness of the silicone (20) is 30-40.

2. The medical thermal mat of claim 1, **characterized in that** the conductive material (34) is configured as component of a heating foil (22).

3. The medical thermal mat of claim 1 or 2, **characterized in that** the conductive material (34) is applied to a flat flexible carrier (30) in a finely distributed manner.

4. The medical thermal mat of claim 3, **characterized in that** the flat flexible carrier (30) is a glass fiber tissue (32).

5. The medical thermal mat of anyone of claims 1 through 4, **characterized in that** the conductive material (34) is a silver-coal-dust-mixture.

6. The medical thermal mat of anyone of claims 1 through 5, **characterized in that** the flat conductive material (34) is covered on both sides with an isolating, flexible plastic foil (40, 42).

7. The medical thermal mat of anyone of claims 1 through 6, **characterized in that** the mat (12) comprises a smooth, pore-free surface (13).

8. The medical thermal mat of anyone of claims 1 through 7, **characterized in that** the mat (12) is connected to a control device (16) via same the conductive material (34) can be electrically supplied and parametered.

9. The medical thermal mat of anyone of claims 1 through 8, **characterized in that** the conductive material (34) can be operated with low voltage.

10. The medical thermal mat of anyone of claims 2 through 9, **characterized in that** several heating foils are provided to be arranged in layers over each other.

## Revendications

1. Natte chauffante médicale flexible pour réchauffer les patients, comportant une natte (12) dans laquelle est intégré un chauffage électrique à résistance (21), le chauffage (21) comportant une matière (34) électriquement conductrice qui se trouve en une quantité telle que des rayons X (44) peuvent traverser la matière (34) sans réflexion, la natte (12) étant en outre en silicone (20) qui enveloppe la matière (34) électriquement conductrice, **caractérisée en ce que** la matière (34) électriquement conductrice s'étend à plat sur toute la surface de la natte (12) et **en ce que** le degré de dureté du silicone (20) est compris entre 30 et 40.

2. Natte chauffante médicale selon la revendication 1, **caractérisée en ce que** la matière (34) électriquement conductrice est réalisée en tant que constituant d'une feuille chauffante (22).

3. Natte chauffante médicale selon la revendication 1 ou 2, **caractérisée en ce que** la matière (34) électriquement conductrice est appliquée finement répartie sur un support flexible (30) plat.

4. Natte chauffante médicale selon la revendication 3, **caractérisée en ce que** le support flexible (30) plat est un tissu en soie de verre (32).

5. Natte chauffante médicale selon l'une des revendications 1 à 4, **caractérisée en ce que** la matière (34) électriquement conductrice est un mélange d'argent et de poudre de carbone.

6. Natte chauffante médicale selon l'une des revendications 1 à 5, **caractérisée en ce que** la matière (34) électriquement conductrice et plate est recouverte sur ses deux faces d'une feuille plastique (40, 42) flexible et isolante.

7. Natte chauffante médicale selon l'une des revendications 1 à 6, **caractérisée en ce que** la natte (12) présente une surface (13) lisse, sans pores.

8. Natte chauffante médicale selon l'une des revendications 1 à 7, **caractérisée en ce que** la natte (12) est reliée à un appareil de commande (16), par lequel la matière (34) électriquement conductrice peut être alimentée électriquement et peut être paramétrée.

9. Natte chauffante médicale selon l'une des revendications 1 à 8, **caractérisée en ce que** la matière (34) électriquement conductrice peut fonctionner avec une basse tension.

10. Natte chauffante médicale selon l'une des revendications 2 à 9, **caractérisée en ce que** sont prévues plusieurs feuilles chauffantes stratifiées les unes sur les autres.
